# EUROPEAN PATENT APPLICATION

(11) **EP 1 201 400 A1**
(43) Date of publication of application: **02.05.2002**
(21) Application number: 00850179.3
(22) Date of filing: 31.10.2000
(51) Int. Cl.: B29C 59/04

(54) **A fluid-pervious fabric and a method of producing it**

(71) Applicant: SCA Hygiene Products AB, 405 03 Göteborg (SE)
(72) Inventor: Hill, Walter, 686 23 Lampertheim (DE); von Paleske, Peter, 672 71 Obersulzen (DE)
(74) Representative: Egeröd, Lisbeth

(57) **Abstract**

A method of producing a fluid-pervious fabric for imparting a pattern to a fibre web, wherein said fabric (101; 201) is brought into a press nip (105) between a rotating embossing roll (106) having embossing means (107) on its outer surface and a support (108). Said fabric comprises at least two zones as seen in the z-direction, a first zone (101a;201a) and a second zone (101b,201b), wherein said first zone comprises a first polymer material having a first glass transition temperature and said second zone comprises a second polymer material having a second glass transition temperature which is higher than that of said first polymer material. The fluid-pervious fabric (101;201) is heated to a temperature close to or slightly below the first glass transition temperature and below the second glass transition temperature before and/or during passage through said press nip (105), wherein the embossing means (107) produces impressions (110, 210) in the surface (102;202) of the fabric, said impressions extending into said said first zone (101a;201a) but not or only slightly extending into said second zone (101b;201b).

## Description

### Technical field

The present invention refers to a method of producing a fluid-pervious fabric for imparting a pattern to a fibre web, wherein said fabric is brought into a press nip between a rotating embossing roll having embossing means on its outer surface and a support. Furthermore, the invention relates to a fluid-pervious fabric, e.g. a wire or a felt, produced by means of the method. The fluid-pervious fabric according to the invention is primarily intended for use as a patterning or shaping fabric in a through-air dryer, but can also be used for wet-forming, foam-forming, air-laying, hydroentangling and/or aperturing in order to impart a desired pattern to a fibre web. The fibre web patterned by means of the fluid-pervious fabric is e.g. a paper web, a nonwoven web, or another fibre web for use in absorbent articles such as toilet paper, handwipes, industrial wipes, diapers, napkins, etc.

### Background of the invention

For different reasons, it can be desirable to create special patterns or indicia in a fibre web. Such patterns can be utilised in order to improve the aesthetic or tactile perception a user gets when observing, handling, or using an absorbent article comprising the fibre web. Suitable patterns can also be utilised in order to improve or enhance the physical properties, such as absorption properties, of an absorbent article.

Examples of articles where patterns of the above-mentioned type have been used previously are toilet paper, handwipes, industrial wipes, diapers, napkins, etc.

The patterning of fibre webs can be performed in a number of previously well-known ways, and can take place e.g. when forming, hydroentangling, pressing, shaping, drying or embossing a fibre web.

One previously well-known way of patterning a fibre web is to utilise a forming wire having metal threads or the like inserted into the wire structure in a desired pattern.

During the forming, the wire will create so-called watermarks in the resulting fibre web.

Furthermore, it has been suggested previously to subject a papermaking wire or felt to an embossing process, wherein a desired pattern is imprinted by means of compressing the internal wire or felt structure in certain areas and leaving the structure unaffected in surrounding areas. Such an embossed fabric can be brought into contact with the fibre suspension or fibre web during the forming, pressing, drying, etc., in order to impart a pattern to the resulting fibre web.

GB-A-1,447,933 discloses a wire for formation and dewatering of paper carrying watermarks. The wire carries regularly spaced embossings to achieve the watermarks. The wire is preferably a bronze wire, but other material such as plastic, may be used. The wire can also be made up of two or more superposed wires, e g one thinner and finer embossed paperside wire and a coarser wire for stability.

GB-A-2,306,178 discloses an embossed forming wire for making watermarks in paper, especially security paper. Is is not mentioned of which material the wire is made.

EP-A-0 394 134 discloses a drying felt or fabric for making paper with marks looking like watermarks. The felt/fabric is heat embossed by means of a heated embossing tool.

Another previously known way of patterning a fibre web is to utilise a forming wire, a press felt or a drying fabric in which portions of the water-pervious wire structure have been partially or completely blocked by a chemical binder or the like.

Accordingly, the international patent application WO 98/53138 discloses a macroscopically mono-planar paper-making belt for a paper machine. The belt has a resinous framework with a web-side surface defining an X-Y-plane, a back-side surface opposite the web-side surface, a Z-direction perpendicular to the X-Y-plane, and discrete deflection conduits extending between the web-side and backside surfaces.

Each conduit has an axis and walls, the axes of at least some of the conduits forming acute angles with the Z-direction.

According to WO 98/53138, the paper-making belt is made by means of disposing liquid photosensitive resin in a forming unit in order to form a coating having opposite surfaces and a pre-selected thickness. The forming unit is disposed in a first direction such that there is an acute angle between a first surface of the coating and the first direction, whereafter a mask having opaque regions and transparent regions defining a pre-selected pattern is positioned between the first surface and an apparatus for generating curing radiation. The unshielded portions but not the shielded portions of the coating are cured by exposing the coating to radiation via the mask in order to form a partially-formed belt. All uncured liquid photosensitive resin is subsequently removed from the belt leaving a hardened resinous structure defining the claimed belt. In WO 98/53138, also a paper web and a process of producing such a web by means of utilising the paper-making belt is disclosed.

Furthermore, it is previously known to impart a pattern to a fibre web in a through-air drying process by means of bringing the fibre web into contact with a special patterning or shaping fabric during the drying, or by means of drying the fibre web between two such fabrics.

Accordingly, the patent publication US 5,893,965 discloses a process for making a paper sheet, wherein a web is transferred from a forming wire to a papermaking belt preferably having deflection conduits. The web is overlaid with a flexible sheet of material such that the web is disposed intermediate the sheet of material and the papermaking belt. The flexible sheet has an air permeability less than the papermaking belt, and is preferably air-impermeable. An application of a fluid pressure differential to the sheet of material causes deflection of at least a portion of the sheet of material towards the papermaking belt and, preferably, deflection of at least a portion of the web into the conduits of the papermaking belt and water removal from the web through the conduits of the papermaking belt.

According to US 5,893,965, the disclosed process makes it possible to produce a paper sheet with e.g. a pattern of protruding domes, but with a more uniform basis weight and density distribution throughout the general plane of the web than what has previously been possible.

However, the previously known methods for producing different patterning fabrics, e.g. wires or felts, can be perceived as having some disadvantages.

Forming or shaping wires produced by means of adding materials such as photosensitive resin, can be sensitive to cleaning with wire showers and wire detergents. Accordingly, there is a great risk that such an added material is washed off, so that the patterning ability gradually disappears.

Previously known methods for producing patterning fabrics by means of methods which reduce the fluid permeability in the treated areas of the fabric, will provide forming fabrics which can cause runnability problems, since the obtainable fabric patterns result in fibre webs with local areas of a much lower grammage than the surrounding areas and, consequently, also with a lower strength. These "weak spots" can cause web breaks and impair the runnability of a paper or nonwoven machine, or otherwise cause an inferior product quality due to holes or linting problems.

The previously utilised shaping fabrics in through-air dryers often exhibit a lower air permeability in the fabric areas intended to produce the pattern. This will create undesired "wet spots" in the dried fibre web.

Previously known methods for producing patterning wires or fabrics by means of embossing are often associated with the problem that they cannot provide wires/fabrics which can produce a sufficiently distinct and clearly visible pattern in a patterned fibre web.

### Summary of the invention

Accordingly, a first object of the present invention is to provide a method of producing a fluid-pervious fabric for imparting a pattern to a fibre web, which fabric can impart a very distinct and clearly visible pattern to a fibre web. The fibre web should be produced with an excellent quality and runnability, since the resulting pattern in the fibre web does not have any weak and wet spots, and since the fluid-pervious fabric exhibits an excellent durability which reduces downtime in the fibre web production. The method should be adapted to produce fluid-pervious fabrics comprising two or more layers, such as a reinforcing layer and an imprinting layer. These objects have according to the invention been provided by the method stated in claim 1 and the dependant claims 2-11.

A further object of the present invention is to provide a fluid-pervious fabric obtained by means of the method according to the invention. This further object is achieved with a fabric in accordance with claim 12 and the dependant claims 13-21.

### Brief description of the drawings

In the following, the present invention will be described in greater detail with reference to the attached drawings, in which
Fig. 1 schematically illustrates an embodiment of the method according to the invention,
Fig. 2 schematically illustrates a section through a fluid-pervious fabric according to a first embodiment of the invention,
Fig. 3 schematically illustrates a section through a fluid-pervious fabric according to a second embodiment of the invention.

### Detailed description of preferred embodiments

The method according to the invention is intended for producing a fluid-pervious fabric 101; 201 for imparting a three-dimensional pattern to a fibre web. The fluid-pervious fabric comprises a first surface 102; 202, a second surface 103; 203 opposite the first surface, and a fabric structure 104; 204 comprising a plurality of channels (not shown) providing fluid permeability between the first and the second surfaces. The fluid-pervious fabric can be of any suitable type, e.g. a papermaking wire of a plastic material, a papermaking felt comprising synthetic polymer fibres, or another suitable fluid-pervious membrane.

The fluid-pervious fabric comprises at least two zones in the z-direction, for example in the form of layers, a first layer101a;201a facing said first surface 102;202 and a second layer 101b;201b facing said second surface 103;203. The first layer comprises a first polymer material having a first glass transition temperature and said second layer comprises a second polymer material having a second glass transition temperature which is higher than that of said first polymer material. Said zones need not be different distinct layers, but can be different zones in the thickness direction (z-direction) of the fabric, said zones being integrated with each other.

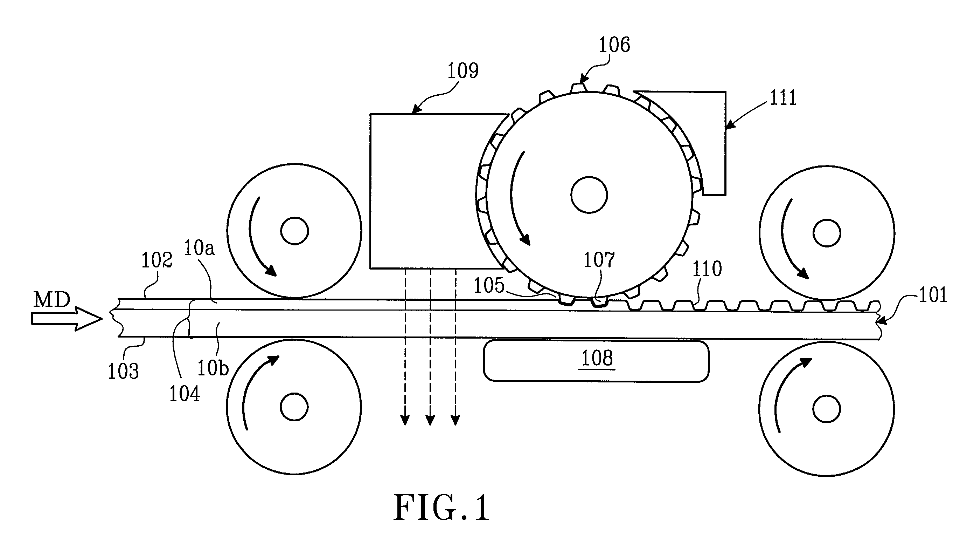

According to Fig. 1 the fluid-pervious fabric according to the invention given a three-dimensional structure by being brought into a press nip 105 between a rotating embossing roll 106 having embossing means 107 on its outer surface and a support 108. The support 108 is constituted of a press shoe or a rotating counter roll which together with the embossing roll forms the press nip.

Heating means 109 is provided immediately before the embossing roll 106 for heating the fluid-pervious fabric 101 to a temperature close to or slightly below the first glass transition temperature and below the second glass transition temperature. Said heating means can also, but need not, heat the embossing roll 106. The heating means 109 is arranged to heat the fluid-pervious fabric 101 to a temperature close to or slightly below the first glass transition temperature and below the second glass transition temperature. Said heating means 109 can be a hot air unit, an infra-red heater, a bath with hot oil, glycerin etc. When the thus heated fabric 101 passes through the press nip 105 the embossing means 107 will cause impressions 110 in the fabric structure101 in the Z-direction in the surface 102 thereof facing the embossing roll 106. The fabric material in the impression 110 will deform and at least to some extent melt and form deformed/ melted areas 110'. Thereafter, the fluid-pervious fabric 101 is cooled down to a temperature lower than the first glass transition temperature in order to render the deformation in the Z-direction permanent. Applicating means 111 may be provided for applying a release agent on the embossing roll 106. The release agent can for example be silicone oil.

The impressions 110 will extend into said first layer 101a but will not extend into said second layer 101b to any substantial extent, since the glass transition temperature of the polymer in the second layer 101b will not be reached. The fluid permeability will be at least slightly reduced in the area of the deformed/melted areas 110' of the impressions 110 as compared to the fluid permeability of the parts of the fabric intermediate the impressions. A totally blocked fluid permeability in the deformed/melted areas 110' of the impressions 110 may also be provided if desired.

The heating of the fabric 101 can alternatively take place solely by means of a heated embossing roll 106, i e without preheating the fabric 101 before entering the press nip 105.

In case the embossing of the first layer 101a of the fabric 101 is performed at a temperature slightly below the glass transition temperature of the polymer material of the first layer 101a the embossing of the fabric structure will cause only a deformation of the structure without any substantial melting of the polymer material of the first layer. By this the fluid permeability will be only slightly reduced in the area of the impressions 110.

The fluid-pervious fabric can be a wire constituted of a plurality of wire strands. The wire strands of the first layer 101a, the imprinting layer, is according to one embodiment interwoven with the wire strands of the second layer 101b, the reinforcement layer. In this case it is not necessary that the wire strands of the first and second layers 101a and b are fused together at the impression sites. It is herewith avoided that the reinforcement strands of the second layer are damaged by the embossment in the press nip 105.

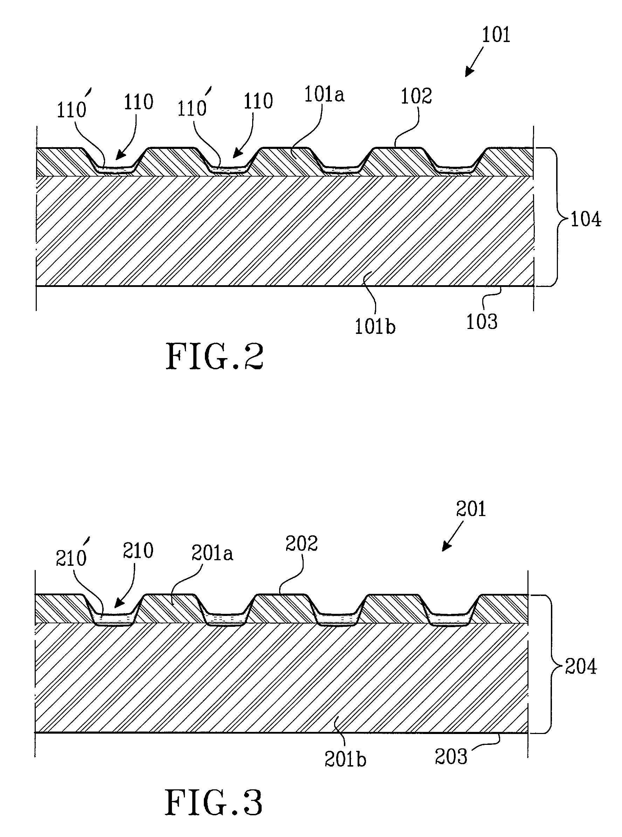

According to another embodiment a double- or multilayer wire structure is produced from two or more separate wire layers, for example a coarse mesh support screen and a fine mesh dewatering screen. The two or more screens are during the embossing in the press nip fused together at the impression sites as the fabric passes through the press nip between the embossing roll 106 and the support 108. This is illustrated in Fig. 3, in which the first and second layers 201a and 201b are fused together at the impression sites 210. Also in this case the glass transition temperature of the polymer material comprised in the first layer 201a is lower than that of the polymer material comprised in the second layer 201b. The wire strands of one or both layers can comprise bicomponent fibers, having a core with a higher melting point and a shell with a lower melting point. In a preferred embodiment the second layer 201b comprises bicomponent fibers having a shell with a melting point close to that of the first layer 201a, in order to provide a secure connection between the two layers at the impression sites.

The fluid-pervious fabric according to the invention may also be a felt constituted of a plurality of felt fibres. Analogous to what is described above with respect to wires, the two or more layers may be united to each other before entering the press nip 105, at which the felt fibers of the first and second layers 101a and b are preferably not fused together at the impression sites (Fig. 2). Alternatively the two layers are united by the embossing that takes place in the press nip by fusing together the felt fibers of the two layers 201a and b (Fig. 3).

The fibre web imprinted by the imprinting fabric according to the invention can be of any suitable type, e.g. a paper web, a board web, a nonwoven web, or any other fibre web. Furthermore, the fibres constituting the fibre web can be of any suitable type, e.g. cellulose pulp fibres and/or natural or manmade fibres based on natural or synthetic polymers, native or recycled.

The imprinting or patterning of fibre webs can be performed in a number of well-known ways, and can take place e.g. when forming, hydroentangling, pressing, shaping, drying or embossing a fibre web.

The fibre web has advantageously been wet-formed or foam-formed. However, it is also conceivable with embodiments in which the fibre web has been air-laid.

The fabric is mainly intended for use as a wet-forming fabric, or as a transfer or drying fabric in a through-air dryer (TAD).

The fabric can also be a felt intended for use as a pressing fabric or as a combined forming/pressing fabric in a Crescent former.

The present invention should by no means be regarded as being limited to what has been described above in connection with the different embodiments, or to what is shown in the attached drawings, but the scope of the invention is defined in the appended claims.

## Claims

1. A method of producing a fluid-pervious fabric for imparting a pattern to a fibre web, said fabric having a first surface (102; 202), a second surface (103; 203) opposite the first surface, and a fabric structure (104; 204) comprising a plurality of channels providing fluid permeability between the first and the second surfaces, wherein said fabric (101; 201) is brought into a press nip (105) between a rotating embossing roll (106) having embossing means (107) on its outer surface and a support (108),
**characterized in**
**that** said fabric comprises at least two zones as seen in the z-direction, a first zone (101a;201a) facing said first surface (102;202) and a second zone (101b;201b) facing said second surface (103;203), wherein said first zone comprises a first polymer material having a first glass transition temperature and said second zone comprises a second polymer material having a second glass transition temperature which is higher than that of said first polymer material,
**that** the fluid-pervious fabric (101;201) is heated to a temperature close to or slightly below the first glass transition temperature and below the second glass transition temperature before and/or during passage through said press nip (105), wherein the embossing means (107) produces impressions (110, 210) in the surface (102;202) of the fabric facing said embossing roll (106) in an embossing pattern corresponding to that of said embossing means (107), said impressions extending into said said first zone (101a;201a) but not or only to a slight extent extending into said second zone (101b;201b).

2. A method as claimed in claim 1, **characterized in**
**that** that the embossing roll (106) is heated to a temperature close to or slightly below said first glass transition temperature.

3. A method as claimed in claim 1 or 2, **characterized in**
**that** heating means (109) are provided before said press nip (105) and heat the fluid-pervious fabric (101) to a temperature close to or slightly below the first glass transition temperature.

4. A method according to any of claims 1-3,
**characterized in that** the support (108) is constituted of a press shoe or a rotating counter roll which together with the embossing roll forms the press nip.

5. A method according to any one of claims 1 - 4,
**characterized in that** the fluid-pervious material (101; 201) is a wire constituted of a plurality of wire strands.

6. A method according to claim 5,
**characterized in that** said first and second zones (101a,b; 201a,b) constitute first and second layers in which the wire strands of the first layer (101a;201a) are interwoven with the wire strands of the second layer (101b;201b).

7. A method according to claim 5,
**characterized in that** said first and second zones (201a,b) constitute first and second layers in which the wire strands of the first and second layers are fused together (210') at the impression sites (210) as the fabric passes through the press nip between the embossing roll (106) and support (108).

8. A method according to any of claims 5-7,
**characterized in that** the first layer (101a;201a) is a fine mesh dewatering screen and the second layer (101b;2016) is a coarse mesh support screen.

9. A method according to any one of claims 1 - 4,
**characterized in that** the fluid-pervious fabric is a felt constituted of a plurality of felt fibres.

10. A method according to any one of the claims 5-9,
**characterized in that** at least a portion of said wire strands or said felt fibres of said first layer (101a;201a) and/or said second layer (101b;2016) are bicomponent fibers having a polymeric core and a polymeric sheath, at which the polymeric core has a higher melting temperature than the polymeric sheath.

11. A method according to any one of the preceding claims,
**characterized in that** applicating means (111) are provided and apply a release agent on the embossing roll (108).

12. A fluid-pervious fabric for imparting a pattern to a fibre web, said fabric having a first surface (102; 202), a second surface (103; 203) opposite the first surface, and a fabric structure (104; 204) comprising a plurality of channels providing fluid permeability between the first and the second surfaces,
**characterized in that** said fabric comprises at least two zones as seen in the z-direction, a first zone (101a;201a) facing said first surface (102;202) and a second zone (101b;201b) facing said second surface (103;203), wherein said first zone comprises a first polymer material having a first glass transition temperature and said second zone comprises a second polymer material having a second glass transition temperature which is higher than that of said first polymer material, that the fluid-pervious fabric (101; 201) exhibits impressions (110;210) extending into said said first zone (101a;201a) but not or only to a slight extent extending into said second zone (101b;201b).

13. A fluid-pervious fabric according to claim 12,
**characterized in that** the fluid-pervious material (101; 201) is a wire constituted of a plurality of wire strands.

14. A method according to claim 13,
**characterized in that** said first and second zones (101a,b; 201a,b) constitute first and second layers in which the wire strands of the first layer (101a;201a) are interwoven with the wire strands of the second layer (101b;201b).

15. A fluid-pervious fabric according to claim 13 or 14,
**characterized in that** wherein the wires of said first and second layers are united by thermal bonds at the impression sites (210).

16. A fluid-pervious fabric according to any of claims12-15,
**characterized in that** the first layer (101a;201a) is a fine mesh dewatering screen and the second layer (101b;201b) is a coarse mesh carrier screen.

17. A fluid-previous fabric according to any of claims 13-16,
**characterized in that** the wire is intended for use as a wet-forming fabric, or as a transfer or drying fabric in a through-air dryer (TAD).

18. A fluid-pervious fabric according to claim 12 or 13,
**characterized in that** the fluid-pervious fabric is a felt constituted of a plurality of felt fibres.

19. A fluid-pervious fabric according to claim 18,
**characterized in that** the felt is intended for use as a pressing fabric or as a combined forming/pressing fabric in a Crescent former.

20. A fluid-pervious fabric according to claim 18,
**characterized in that** the felt fibres of said first (201a) and second layers (201b) are united by thermal bonds at the impression sites (210).

21. A fluid-pervious fabric according to any one of the claims 12-20,
**characterized in that** at least a portion of said wire strands or said felt fibres of said first layer (101a;201a) and/or said second layer (101b;201b) are bicomponent fibers having a polymeric core and a polymeric sheath, at which the polymeric core has a higher melting temperature than the polymeric sheath.
